# EUROPÄISCHE PATENTSCHRIFT

(11) **EP 0 557 787 B1**
(45) Veröffentlichungstag und Bekanntmachung des Hinweises auf die Patenterteilung: **24.05.2000**
(21) Anmeldenummer: 93101999.6
(22) Anmeldetag: 09.02.1993
(51) Int. Cl.: C07D 233/60, A61K 31/415

(54) **Imidazolylphenolderivate, diese enthaltende Arzneimittel sowie ein Verfahren zur Herstellung dieser Verbindungen und Arzneimittel**
Imidazolylphenol derivatives, medicaments containing them and a process for the preparation of these compounds and medicaments
Dérivés d'imidazolylphénol, médicaments les contenant et un procédé de préparation de ces composés et médicaments

(30) Priorität: 17.02.1992 DE 4204686
(43) Veröffentlichungstag der Anmeldung: 01.09.1993
(73) Patentinhaber: Grünenthal GmbH, 52078 Aachen (DE)
(72) Erfinder: Ammann, Joachim, Dr., W-5100 Aachen (DE); Haurand, Michael, Dr., W-5190 Stolberg (DE); Geist, Cornelia, Dr., W-5100 Aachen (DE); Englberger, Werner, Dr., W-5190 Stolberg (DE); Zimmer, Oswald, Dr., W-5102 Würselen (DE)

(56) Entgegenhaltungen:
- EP-A- 0 232 954
- EP-A- 0 292 699
- EP-A- 0 384 594
- EP-A- 0 452 908
- WO-A-90/12008
- FR-A- 2 444 031

## Beschreibung

Polyungesättigte höhere Fettsäuren, beispielsweise Arachidonsäure, dienen im Stoffwechsel des Menschen und von Säugetieren als Substrate für die enzymatisch katalysierte Bildung physiologisch und pathophysiologisch bedeutsamer Eicosanoide, wie Prostaglandine, Thromboxane, Prostacyclin und Leukotriene. Dabei wird die Bildung von Prostaglandinen, Thromboxanen und Prostacyclin durch das Enzym Cyclooxygenase, die Leukotrienbildung durch 5-Lipoxygenase eingeleitet.

Thromboxan A2, eine extrem instabile und biologisch aktive Verbindung, die neben der Kontraktion der glatten Muskulatur eine starke Aggregation der Blutplättchen bewirkt, wird in zahlreichen Zellen, beispielsweise menschlichen Blutplättchen, aus Prostaglandin-Endoperoxiden in Gegenwart von Thromboxansynthase gebildet (Biochem. Biophys. Res. Commun. 80, 236 (1978)). Von einigen 1-substituierten Imidazolen ist bekannt, daß sie die Aktivität der Thromboxansynthase (in der Literatur häufig auch als Thromboxansynthetase bezeichnet) und damit die Bildung von Thromben hemmen.

Leukotriene sind wichtige Mediatoren bei Entzündungs- und Immunreaktionen und spielen eine bedeutende Rolle bei der Entstehung von lebensbedrohlichen Situationen, beispielsweise beim anaphylaktischen oder auch septischen Schock, aber auch bei allergischen Reaktionen, Bronchokonstriktionen und Asthma. Die Vielzahl der mit Leukotrienen in Zusammenhang stehenden unerwünschten Effekte hat dazu geführt, daß zahlreiche Verbindungen hinsichtlich ihrer Hemmwirkung gegenüber 5-Lipoxygenase untersucht wurden. So sind beispielsweise aus EP 292 699 und WO 90/12008 phenyl-, naphthyl- und thienylsubstituierte N-Hydroxyharnstoffderivate als 5-Lipoxygenasehemmer bekannt.

Mit keinem der bekannten 5-Lipoxygenasehemmer und Thromboxansynthasehemmer ist es jedoch möglich, gleichzeitig 5-Lipoxygenase und Thromboxansynthase zu hemmen, mit der Folge, daß zwei Substanzen appliziert werden müssen, wenn sowohl 5-Lipoxygenase als auch Thromboxansynthase gehemmt werden sollen. Die der Erfindung zugrundeliegende Aufgabe bestand somit in der Bereitstellung von toxikologisch unbedenklichen, stabilen Verbindungen, die sowohl 5-Lipoxygenase als auch Thromboxansynthase hemmen.

Es wurde gefunden, daß bisher nicht bekannte Imidazolylphenolderivate die an sie gestellten hohen Anforderungen erfüllen.

Gegenstand der Erfindung sind dementsprechend 3- oder 4-(1H-Imidazol-1-yl)phenolderivate der allgemeinen Formel I 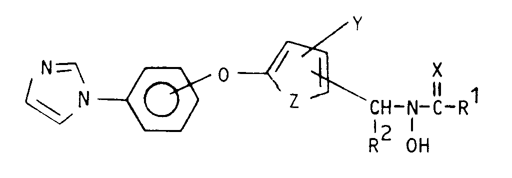 in der R¹ NH₂ oder CH₃, R² H oder CH₃, X O oder S, Y H, OH oder OCH₃ und Z -CH=CH-, O oder S bedeuten, und/oder deren Salze.

Steht in der allgemeinen Formel I der Rest R² für die Methylgruppe, so tritt an dem diesen Rest tragenden Kohlenstoffatom ein Asymmetriezentrum auf. Zum Gegenstand der Erfindung gehören dementsprechend auch Razemate und optisch aktive Formen der Verbindungen der allgemeinen Formel I.

Die Substituenten Y und 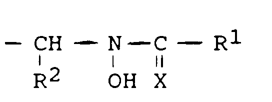 in der allgemeinen Formel I können sich im Phenyl-, Thienyl- oder Furanylrest in jeder beliebigen Stellung befinden.

Imidazolylphenolderivate und/oder deren Salze, in denen der Rest R² Wasserstoff bedeutet, werden bevorzugt. Imidazolylphenolderivate und/oder deren Salze, in denen Z -CH=CH- bedeutet, insbesondere N-Hydroxy-N-[4-[4-(1H-imidazol-1-yl)phenoxy]phenylmethyl]harnstoff, das Imidazoliumchlorid dieser Verbindung, N-Hydroxy-N-[4-[4-(1H-imidazol-1-yl)phenoxy]phenylmethyl]acetamid, N-Hydroxy-N-[3-[4-(1H-imidazol-1-yl)phenoxy]phenylmethyl]harnstoff, N-Hydroxy-N-{1-[4-[4-(1H-imidazol-1-yl)phenoxy]phenyl]ethyl}harnstoff, N-Hydroxy-N-[4-[4-(1H-imidazol-1-yl)phenoxy]phenylmethyl]thioharnstoff, N-Hydroxy-N-[4-[3-(1H-imidazol-1-yl)phenoxy]phenylmethyl]harnstoff und N-Hydroxy-N-[3-[4-(1H-imidazol-1-yl)phenoxy]-6-methoxy-phenylmethyl]harnstoff, Imidazolylphenolderivate und/oder deren Salze, in denen Z S bedeutet, insbesondere N-Hydroxy-N-[5-[4-(1H-imidazol-1-yl)phenoxy]thien-2-yl-methyl]harnstoff sowie Imidazolylphenolderivate und/oder deren Salze, in denen Z O bedeutet, insbesondere N-Hydroxy-N-[5-[4-(1H-imidazol-1-yl)-phenoxy]furan-2-yl-methyl]harnstoff, sind besonders geeignete Verbindungen.

Die erfindungsgemäßen Imidazolylphenolderivate und/oder deren Salze hemmen selektiv 5-Lipoxygenase und Thromboxansynthase, während die Aktivität der 12-Lipoxygenase sowie der Cyclooxygenase nicht beeinflußt wird. Die daraus resultierende Hemmung der Bildung der pharmakologisch äußerst wirksamen Arachidonsäuremetabolite 5-Hydroxyeicosatetraensäure(5-HETE), der Leukotriene wie zum Beispiel LTB₄, LTC₄, LTD₄ und LTE₄, sowie Thromboxan A2 verleiht den erfindungsgemäßen Verbindungen zahlreiche physiologisch wertvolle Eigenschaften, beispielsweise antithrombotische, antivasospastische, antivasokonstriktorische, aggregationshemmende, antianaphylaktische, antiasthmatische, antiallergische, antiphlogistische, blutdrucksenkende, durchblutungsfördernde (coronarer und cerebraler Kreislauf) sowie antipsoriatische Wirkungen. Desweiteren wird durch die erfindungsgemäßen Verbindungen die Aktivierung, die Aggregation und damit verbunden der Verbrauch an Thrombozyten und Leukozyten sowie die Aktivierung von Makrophagen, beispielsweise Alveolearmakrophagen und Kupfferzellen, und die Aktivierung von glatten Muskelzellen in Geweben, Venen und Arterien gehemmt. Da die erfindungsgemäßen Verbindungen der allgemeinen Formel I chemisch und metabolisch für eine therapeutische Anwendung stabil und lagerfähig sind, eignen sie sich zum Einsatz als Arzneimittel, beispielsweise als Thrombozytenaggregationshemmer, Vasodilatator, Antiallergikum, Antianaphylaktikum, Antiphlogistikum, Antiasthmatikum, Antihypertensivum, als Mittel zur Prophylaxe oder Therapie von ischämischem Herzinfarkt und/oder zur Behandlung von Störungen coronarer und/oder cerebraler Gefäße.

Die Imidazolylphenolderivate und/oder deren Salze sind toxikologisch unbedenklich, so daß sie als solche in geeigneten pharmazeutischen Zubereitungen an Mensch und Tier verabreicht werden können.

Weiterer Erfindungsgegenstand sind dementsprechend Arzneimittel, die als Wirkstoff wenigstens ein 3- oder 4-(1H-Imidazol-1-yl)phenolderivat der allgemeinen Formel I und/oder wenigstens ein entsprechendes Salz enthalten.

Die an den Patienten zu verabreichende Wirkstoffmenge variiert in Abhängigkeit vom Gewicht des Patienten, von der Applikationsart, der Indikation und dem Schweregrad der Erkrankung. Unter Berücksichtigung dieser Faktoren liegt der Wirkstoffgehalt pro Einzeldosis im allgemeinen zwischen 0,01 und 1000 mg, wobei Zubereitungsformen für die parenterale Applikation 0,1 bis 1000 mg Wirkstoff, Zubereitungsformen für die topische und inhalative Anwendung 0,01 bis 100 mg Wirkstoff und Zubereitungsformen für die orale Applikation 1 bis 1000 mg Wirkstoff enthalten können.

Aufgrund der sehr guten Wasserlöslichkeit der Imidazolylphenolderivatsalze eignen sich diese insbesondere zur topischen und inhalativen Anwendung sowie zur parenteralen Applikation. Für diese Applikationsformen eignen sich Lösungen, Suspensionen, leicht rekonstituierbare Trockenzubereitungen sowie für topische und inhalative Anwendungen auch Sprays.

Darüber hinaus eignen sich für viele prophylaktische oder therapeutische Anwendungen auch oral anwendbare Zubereitungsformen der erfindungsgemäßen Verbindungen der allgemeinen Formel I, wie Tabletten, Dragees, Kapseln, Granulate, Tropfen, Säfte und Sirupe, aber auch Suppositorien sowie perkutane Applikationszubereitungen, beispielsweise Wirkstoffe in einem Depot in gelöster Form oder gegebenenfalls unter Zusatz von die Hautpenetration fördernden Mitteln enthaltende Pflaster. Vorteilhaft werden diese oral, rektal oder perkutan anwendbaren Zubereitungsformen so hergestellt, daß daraus der Wirkstoff verzögert freigesetzt wird, um so über einen längeren Zeitraum, beispielsweise von 24 Stunden, eine gleichmäßige Versorgung des Patienten mit dem Wirkstoff zu gewährleisten.

Alle vorstehend aufgeführten pharmazeutischen Zubereitungsformen sind an sich bekannt, und da die erfindungsgemäßen Verbindungen der allgemeinen Formel I chemisch stabil sind, wirft ihre Einarbeitung in diese Zubereitungsformen für den Fachmann keinerlei Probleme auf. Bei der erfindungsgemäßen Herstellung dieser Arzneimittel muß selbstverständlich mit der üblichen Sorgfalt bei Auswahl der Hilfsstoffe, beispielsweise Trägermaterialien, Lösungsmittel, Verdünnungsmittel, Farbstoffe, Geschmackskorrigenzien, Bindemittel und Tablettensprengstoffe, vorgegangen werden, und insbesondere bei der Herstellung von parenteral anzuwendenden Zubereitungsformen ist auf Sterilität und, sofern diese in flüssiger Form vorliegen, Isotonie zu achten.

Weiterer Erfindungsgegenstand ist ein Verfahren zur Herstellung von 3- oder 4-(1H-Imidazol-1-yl)phenolderivaten der allgemeinen Formel I 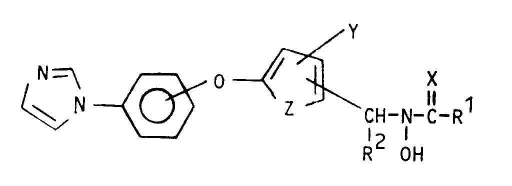 in der R¹ NH₂ oder CH₃, R² H oder CH₃, X O oder S, Y H, OH oder OCH₃ und Z -CH=CH-, O oder S bedeuten, und/oder deren Salzen, welches dadurch gekennzeichnet ist, daß man eine Carbonylverbindung der allgemeinen Formel II

R³-Aryl

in der R³ Halogen, NO₂ oder OH und Aryl 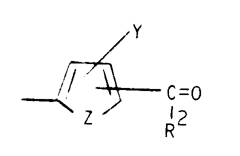 bedeuten, in Gegenwart eines Lösungsmittels, gegebenenfalls katalytischer Mengen an Kupfer und/oder Kupfer-I-salzen und, wenn R³=OH ist, nach Umsetzung mit einem Sulfochlorid, mit 3-(1H-Imidazol-1-yl)phenol oder 4-(1H-Imidazol-1-yl)phenol und/oder einem Alkalisalz eines dieser Phenole zu einer Verbindung der allgemeinen Formel III 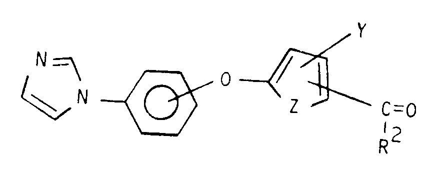 umsetzt, die Verbindung der allgemeinen Formel III in Gegenwart eines Lösungsmittels mit Hydroxylamin und/oder einem seiner Salze mit oder ohne Zusatz einer Base in das Oxim der allgemeinen Formel IV 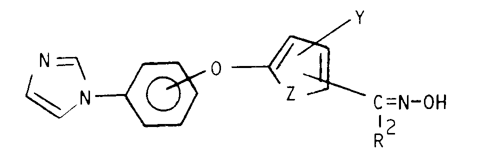 überführt, anschließend das erhaltene Oxim mit boranhaltigen Reduktionsmitteln in Gegenwart einer Säure zum Hydroxylamin der allgemeinen Formel V 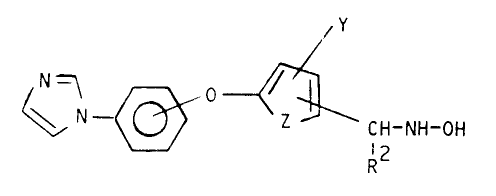 reduziert, das erhaltene Hydroxylamin in das Imidazolylphenolderivat der allgemeinen Formel I überführt entweder
A. durch Umsetzung mit einem Alkalicyanat oder einem Alkalithiocyanat oder
B. durch Umsetzung mit Trimethylsilylisocyanat oder Trimethylsilylisothiocyanat und anschließende Abspaltung der Trimethylsilylgruppe oder
C. durch Umsetzung mit einem Acetylierungsmittel und anschließende selektive basische Hydrolyse der O-Acetylgruppe der erhaltenen Bis-acetylverbindung
und danach gegebenenfalls aus dem Imidazolylphenolderivat der allgemeinen Formel I mit einer Säure ein Salz herstellt.

Verbindungen der allgemeinen Formel III werden vorzugsweise durch Umsetzung einer Carbonylverbindung der allgemeinen Formel II

R³-Aryl

in der R³ Cl, Br oder J und Aryl 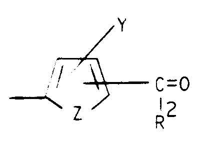 bedeuten, hergestellt. Zu diesem Zweck wird 3-(1H-Imidazol-1-yl)phenol oder 4-(1H-Imidazol-1-yl)phenol, gegebenenfalls gelöst in einem polaren Lösungsmittel oder Lösungsmittelgemisch, mit etwa 1 bis 5 Äquivalenten einer oder mehrerer Basen wie Natrium- und/oder Kaliumhydroxyd, Natrium- und/oder Kaliumcarbonat, und/oder Natriumalkoholat, beispielsweise Natriummethylat, in das entsprechende Alkalisalz überführt und anschließend in Gegenwart eines polaren Lösungsmittels oder Lösungsmittelgemisches sowie in Gegenwart katalytischer Mengen an aktiviertem Kupferpulver und/oder Kupfer-I-salzen, beispielsweise Kupfer-I-halogeniden, nach Zugabe einer Carbonylverbindung der allgemeinen Formel II in einer Menge - bezogen auf eingesetzte Menge an (1H-Imidazol-1-yl)phenol - von etwa 1 bis 2 Äquivalenten auf eine Temperatur zwischen 50 und 160° C erhitzt. Als polare Lösungsmittel eignen sich beispielsweise Dimethylsulfoxid, Dimethylformamid, Alkohole wie Methanol oder Ethanol, Tetrahydrofuran und 1,4-Dioxan. Vorzugsweise werden Dimethylsulfoxid und/oder Dimethylformamid verwendet (H. Meerwein in "Methoden der organischen Chemie", Houben-Weyl 4. Aufl., Bd. 6/3, S. 85 bis 89, Stuttgart 1965).

Eine weitere Möglichkeit, Verbindungen der allgemeinen Formel III herzustellen, besteht darin, 3-(1H-Imidazol-1-yl)phenol oder 4-(1H-Imidazol-1-yl)phenol, das in Gegenwart eines oder mehrerer der oben genannten polaren Lösungsmittel mit einer der oben genannten Basen in das entsprechende Alkalisalz überführt wurde, mit einer Carbonylverbindung der allgemeinen Formel II

R³-Aryl

in der R³ NO₂ und Aryl 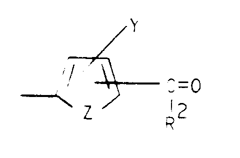 bedeuten, in einer Menge von 0,9 bis 1,0 Äquivalenten bei einer Temperatur zwischen 40 und 160° C umzusetzen (J. Heterocyclic Chem. 18, 1241 (1981)).

Des weiteren lassen sich Verbindungen der allgemeinen Formel III erhalten, indem eine in einem tertiären Amin, insbesondere Pyridin gelöste Carbonylverbindung der allgemeinen Formel II

R³-Aryl

in der R³ OH und Aryl 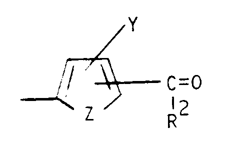 bedeuten, mit etwa 1 bis 2 Äquivalenten eines Sulfochlorides, beispielsweise Benzol-, Toluol-, Methan- oder Trifluormethansulfochlorid, in das entsprechende Sulfonat überführt und anschließend mit einem Äquivalent 3-(1H-Imidazol-1-yl)phenol oder 4-(1H-Imidazol-1-yl)phenol zu einer Verbindung der allgemeinen Formel III umgesetzt wird (H. Meerwein in "Methoden der oganischen Chemie", Houben-Weyl 4. Aufl., Bd. 6/3, S. 89 bis 90, Stuttgart 1965).

Die Verbindungen der allgemeinen Formel III 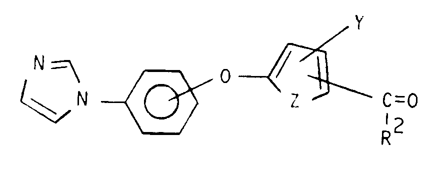 in der R² vorzugsweise H, Y H, OH oder OCH₃ und Z -CH=CH-, O oder S bedeuten, können in bekannter Weise in einem polaren Lösungsmittel oder Lösungsmittelgemisch, beispielsweise Methanol, Ethanol, Tetrahydrofuran und/oder Pyridin, mit 1 bis 2 Äquivalenten Hydroxylamin und/oder einem seiner Salze, beispielsweise Hydroxylammoniumchlorid, in Gegenwart einer oder mehrerer Basen, beispielsweise Pyridin, Triethylamin, Alkalialkoholaten wie Natriummethylat, Kalium-tert.-butylat, Kalium- und/oder Natriumcarbonat und/oder Natriumacetat bei einer Temperatur zwischen 18 und 25° C in Oxime der allgemeinen Formel IV überführt werden. Anschließend werden die erhaltenen Oxime in einem polaren Lösungsmittel oder Lösungsmittelgemisch, beispielsweise Alkylalkoholen wie Methanol und Ethanol, Tetrahydrofuran und/oder 1,4-Dioxan, mit boranhaltigen Reduktionsmitteln, vorzugsweise mit einem Boran-Amin-Komplex, beispielsweise Boran-Pyridin-Komplex, und/oder mit dem Boran-Tetrahydrofuran-Komplex, in Gegenwart einer oder mehrerer Säuren, beispielsweise Salzsäure und/oder Essigsäure, bei Temperaturen zwischen 0 und 5° C zu Hydroxylaminen der allgemeinen Formel V reduziert (J. B. Summers et al., J. Med. Chem. 31 , 1960 (1988)).

Zur Herstellung der 3- und 4-(1H-Imidazol-1-yl)phenolderivate der allgemeinen Formel I, in denen der Rest R¹ NH₂ bedeutet, können die Hydroxylamine der allgemeinen Formel V ohne Isolierung mit 1 bis 6 Äquivalenten Alkalicyanat oder Alkalithiocyanat, beispielsweise Kaliumcyanat oder Kaliumthiocyanat umgesetzt werden. Eine weitere Möglichkeit zur Herstellung der Imidazolylphenolderivate der allgemeinen Formel I besteht darin, die Hydroxylamine der allgemeinen Formel V in an sich bekannter Weise zu isolieren und mit 1 bis 1,4 Äquivalenten Trimethylsilylisocyanat oder Trimethylsilylisothiocyanat in aprotischen Lösungsmitteln wie cyclischen Ethern, beispielsweise Tetrahydrofuran und 1,4-Dioxan, Dimethylsulfoxid und Dimethylformamid, bei Temperaturen zwischen 20 und 80° C umzusetzen. Durch Hydrolyse des intermediär gebildeten Additionsproduktes, beispielsweise mit gesättigter Ammoniumchloridlösung, werden die gewünschten Verbindungen der allgemeinen Formel I erhalten.

Imidazolylphenolderivate der allgemeinen Formel I, in denen der Rest R¹ CH₃ bedeutet, können aus den Hydroxylaminen der allgemeinen Formel V erhalten werden, indem die Hydroxylamine gegebenenfalls ohne vorherige Isolierung mit 2 bis 3,5 Äquivalenten eines Acetylierungsmittels, vorzugsweise mit Essigsäureanhydrid, Essigsäureethylester und/oder Essigsäurechlorid, in die entsprechenden Bis-Acetylverbindungen überführt werden, aus denen durch selektive basische Hydrolyse der O-Acetylgruppe in einem oder mehreren Alkylalkoholen wie Methanol, Ethanol und/oder Isopropanol, als Lösungsmittel bei Temperaturen zwischen 20 und 60° C die Imidazolylphenolderivate der allgemeinen Formel I gebildet werden. Als Basen dienen bei der Hydrolyse beispielsweise Lithium-, Natrium- und/oder Kaliumhydroxid und/oder Natrium- und/oder Kaliumcarbonat, die gegebenenfalls in Form wäßriger Lösungen zugesetzt werden.

Aus den Imidazolylphenolderivaten der allgemeinen Formel I lassen sich die entsprechenden Salze durch Zugabe mindestens äquimolarer Mengen einer oder mehrerer Säuren wie Salzsäure und/oder Schwefelsäure, zu einer wäßrigen Suspension von Imidazolylphenolderivaten herstellen. Die gebildeten Salze können beispielsweise in Gegenwart von Aceton ausgefällt und aus Ethanol umkristallisiert werden oder durch Gefriertrocknung isoliert werden.

### Beispiele

Alle Temperaturangaben sind unkorrigiert.

Die ¹H-NMR-Spektren wurden bei 300 MHz mit dem Gerät AC 300 der Firma Bruker gemessen. Die chemische Verschiebung der spektroskopischen Resonanzdaten ist in ppm angegeben. DMSO-d₆ bedeutet deuteriertes Dimethylsulfoxid.

Als stationäre Phase für die säulenchromatographischen Trennungen wurde Kieselgel 60 mit einer Korngröße von 0,063 bis 0,200 mm (entsprechend 70 bis 230 mesh ASTM) der Firma E. Merck, Darmstadt, eingesetzt.

Kupferpulver wurde nach der in Org. Synthesis, Coll. Vol. II, 446 beschriebenen Methode aktiviert.

### Beispiel 1

### N-Hydroxy-N-[4-[4-(1H-imidazol-1-yl)phenoxylphenylmethyl]harnstoff

### a) 4-[4-(1H-Imidazol-1-yl)phenoxy]benzaldehyd

Eine Suspension aus 40,04 g (0,25 Mol) 4-(1H-Imidazol-1-yl)phenol, 15,2 g (0,11 Mol) getrocknetem Kaliumcarbonat, 1 g Kupfer-I-bromid und 0,5 g aktiviertem Kupferpulver in 100 ml absolutem Dimethylformamid wurde auf 90° C erwärmt und diese innerhalb von 7 Stunden nach und nach mit insgesamt 47,03 g (0,25 Mol) 4-Brombenzaldehyd versetzt. Während der Aldehydzugabe wurde auf 140 bis 145° C aufgeheizt. Nach beendeter Aldehydzugabe wurde auf 20° C abgekühlt, das Reaktionsgemisch mit 1500 ml eiskaltem Essigsäureethylester versetzt und mit 500 ml 1 N wäßriger Natronlauge geschüttelt. Nach Abtrennung ungelöster Bestandteile wurde die organische Phase durch dreimaliges Ausschütteln mit insgesamt 500 ml 1 N wäßriger Natronlauge von Resten an Imidazolylphenol befreit und dreimal mit je 100 ml gesättigter wäßriger Natriumchloridlösung gewaschen. Nach Trocknung über Magnesiumsulfat und Abtrennung des Trockenmittels wurde das Filtrat bei 45° C und 2,6 · 10³ bis 6 · 10³ Pa eingedampft. Der erhaltene Rückstand wurde durch Säulenchromatographie mit Essigsäureethylester gereinigt. Man erhielt 37,37 g (56,6 % der Theorie) kristallinen, gelbbraunen 4-[4-(1H-Imidazol-1-yl)phenoxy]benzaldehyd mit einem Schmelzpunkt von 120 bis 121° C.
- ¹H-NMR (DMSO-d₆):: 7,14 - 7,18 (m, 3H, aromat.);
7,29 - 7,32 (m, 2H, aromat.);
7,74 - 7,77 (m, 3H, aromat.);
7,94 - 7,96 (m, 2H, aromat.),
8,26 (s, 1H, aromat.);
9,95 (s, 1H, CHO)

### b) N-Hydroxy-N-[4-[4-(1H-imidazol-1-yl)phenoxy]phenylmethyl]harnstoff

0,9 g (13 mMol) Hydroxylammoniumchlorid, gelöst in 9 ml absolutem Methanol, wurden mit 13 ml einmolarer (1 N) methanolischer Natriummethylatlösung und anschließend mit einer Lösung von 2,64 g (10 mMol) des nach a) erhaltenen Aldehyds, gelöst in 19 ml absolutem Methanol, versetzt. Nach 60-minütigem Rühren bei 20° C wurde das Reaktionsgemisch im Eisbad abgekühlt und mit 4 ml (40 mMol) Boran-Pyridin-Komplex versetzt. Nach 2 Stunden bei 0° C wurden 5,3 ml (63 mMol) wäßrige konzentrierte Salzsäure und nach einer weiteren Stunde 3,3 ml (30 mMol) wäßrige konzentrierte Salzsäure zugegeben. Nach 24 Stunden bei + 4° C wurde der pH-Wert der Reaktionsmischung unter Kühlung mit 5 ml 10molarer Natronlauge auf 6 eingestellt. Danach wurde tropfenweise mit einer Lösung von 4,9 g (60 mMol) Kaliumcyanat in 10 ml Wasser versetzt und nach 5,5 stündigem Stehenlassen bei 20° C das Reaktionsgemisch bei 45° C und 1,5 · 10³ Pa eingedampft. Der Rückstand wurde mit einem Gemisch aus 50 ml Tetrahydrofuran und 70 ml Essigsäureethylester versetzt und zweimal mit je 20 ml 0,1 molarer Natronlauge und abschließend mit 30 ml gesättigter wäßriger Natriumchloridlösung ausgeschüttelt. Sodann wurde die organische Phase mit Magnesiumsulfat versetzt und nach Abtrennen von Trockenmittel und nach Zugabe von Toluol Lösungsmittel bei 2,6 · 10³ bis 6 · 10³ Pa und 45° C abdestilliert. Nach dem Trocknen des erhaltenen Rückstandes bei einem Druck <13 Pa und Umkristallisieren mit einer Mischung aus 15 ml Methanol und 9 ml absolutem Diethylether wurden 2,18 g (67,2 % der Theorie) farbloser kristalliner N-Hydroxy-N-[4-[4-(1H-imidazol-1-yl)phenoxy]phenylmethyl]harnstoff mit einem Schmelzpunkt von 159° C erhalten.
- ¹H-NMR (DMSO-d₆):: 4,52 (s, 2H, CH₂);
6,35 (s, 2H, NH₂);
6,99 - 7,10 (q, 2H, aromat.);
7,13 - 7,14 (m, 3H, aromat.);
7,32 - 7,34 (q, 2H, aromat.);
7,61 - 7,65 (m, 3H, aromat.);
8,16 (s, 1H, aromat.);
9,37 (s, 1H, OH)

### Beispiel 2

### Imidazoliumchlorid von N-Hydroxy-N-[4-[4-(1H-imidazol-1-yl)phenoxy]phenylmethyl]harnstoff

3,24 g (10 mMol) N-Hydroxy-N-[4-[4-(1H-imidazol-1-yl)phenoxy]phenylmethyl]harnstoff wurden in 10 ml Wasser suspendiert und durch Zugabe von 1,7 ml 6 N-Salzsäure in Lösung gebracht. Das Imidazoliumchlorid wurde durch Zugabe von 150 ml Aceton gefällt, anschließend mit Aceton säurefrei gewaschen und aus 150 ml absolutem Ethanol umkristallisiert. Es wurden 2,85 g (80 % der Theorie) Imidazoliumchlorid mit einem Zersetzungspunkt bei 156°C erhalten.
- ¹H-NMR (DMSO-d₆):: 4,54 (s, 2H, CH₂);
6,37 (s, 1H, NH₂);
7,03 - 7,19 (q, 2H, aromat.);
7,20 - 7,38 (q, 2H, aromat.);
7,80 - 7,84 (m, 2H, aromat.);
7,90 - 7,91 (t, 2H, aromat.);
8,25 - 8,26 (t, 1H, aromat.);
8,33 (d, 1H, aromat.);
9,45 (s, 1H, OH);
9,75 (s, 1H, aromat.);
ca. 16 (s, breit, 1H⁺, aromat.)

### Beispiel 3

### N-Hydroxy-N-[4-[4-(1H-imidazol-1-yl)phenoxy]phenylmethyl]acetamid

Wie in Beispiel 1 angegeben, wurde der nach 1a) hergestellte Benzaldehyd in das Oxim überführt und dieses mit Boran-Pyridin-Komplex zum Hydroxylamin reduziert. Nach Ansäuern mit wäßriger, konzentrierter Salzsäure und Abdestillieren von Methanol bei 45° C und einem Druck zwischen 2,6 · 10³ und 6 · 10³ Pa wurde der wäßrige Rückstand mit dem gleichen Volumen Essigsäureethylester überschichtet, im Eisbad abgekühlt und unter weiterer Kühlung mit 10 N wäßriger Natronlauge ein pH-Wert von 10 eingestellt. Das ausfallende Öl wurde mit Essigsäureethylester extrahiert, der Extrakt über Magnesiumsulfat getrocknet, und nach Zugabe von 1/10 Volumen Toluol bei 1,5 · 10³ Pa und 45° C eingeengt und anschließend bei 40° C und 13 Pa getrocknet. 2,95 g (10,5 mMol) des Rückstandes wurden in 80 ml absolutem Tetrahydrofuran gelöst und bei 0° C unter Rühren mit 5,15 ml (37 mMol) Triethylamin und anschließend mit 2,76 ml (36,2 mMol) Essigsäurechlorid, gelöst in 20 ml absolutem Tetrahydrofuran, versetzt. Nach 30 Minuten wurde das Reaktionsgemisch bei 4 · 10³ Pa eingedampft, der Rückstand in 20 ml absolutem Methanol gelöst, mit 100 ml Wasser versetzt und mit Essigsäureethylester extrahiert. Nach Trocknen und Eindampfen des Extraktes bei 45° C und 4 · 10³ Pa wurden 7,28 g eines gelben Öles erhalten, das in 70 ml Isopropanol gelöst und unter Zugabe einer Lösung von 1,5 g Lithiumhydroxid in 15 ml Wasser innerhalb von 3 Stunden bei 40° C verseift wurde. Nach Zugabe von 200 ml Wasser wurde der pH-Wert auf 7 eingestellt und mit Essigsäureethylester extrahiert. Nach Trocknen mit Magnesiumsulfat und Eindampfen des Extraktes wurden 5,95 g eines hellgelben festen Rückstandes erhalten, der durch Säulenchromatographie mit Tetrahydrofuran gereinigt wurde. Es wurden 3,17 g schwachgelbes N-Hydroxy-N-[4-[4-(1H-imidazol-1-yl)phenoxy]phenylmethyl]acetamid in Form von Kristallen, mit einem Schmelzpunkt von 149° C erhalten.
- ¹H-NMR (DMSO-d₆):: 2,05 (s, 3H, CH₃);
4,67 (s, 2H, CH₂);
7,01 - 7,04 (q, 2H, aromat.);
7,09 - 7,15 (m, 3H, aromat.);
7,29 - 7,32 (q, 2H, aromat.);
7,61 - 7,67 (m, 3H, aromat.);
8,17 (s, 1H, aromat.); 9,89 (s, 1H, OH)

### Beispiel 4

### N-Hydroxy-N-[5-[4-(1H-imidazol-1-yl)phenoxy]thien-2-yl-methyl]harnstoff

Die Herstellung erfolgte analog Beispiel 1. Anstelle von 4-Brombenzaldehyd wurden 47,8 g (0,25 Mol) 5-Bromthiophen-2-aldehyd eingesetzt.

Der Schmelzpunkt von N-Hydroxy-N-[5-[4-(1H-imidazol-1-yl)phenoxy]thien-2-yl-methyl]harnstoff lag bei 141° C.
- ¹H-NMR (DMSO-d₆):: 4,56 (s, 2H, CH₂); 6,44 (s, 2H, NH₂);
6,52 - 6,54 (d, 1H, aromat.);
6,76 - 6,77 (d, 1H, aromat.);
7,09 (s, 1H, aromat.);
7,22 - 7,25 (m, 2H, aromat.);
7,63 - 7,67 (m, 3H, aromat.);
8,18 (s, 1H, aromat.);
9,5 - 10,0 (s, breit, 1H, OH)

### Beispiel 5

### N-Hydroxy-N-[3-[4-(1H-imidazol-1-yl)phenoxy]phenylmethyl]harnstoff

Die Herstellung erfolgte analog Beispiel 1. Anstelle von 4-Brombenzaldehyd wurden 46,3 g (0,25 Mol) 3-Brombenzaldehyd eingesetzt. Der Schmelzpunkt der erhaltenen Verbindung betrug 163° C.
- ¹H-NMR (DMSO-d₆):: 4,53 (s, 2H, CH₂); 6,39 (s, 2H, NH₂);
6,91 - 6,95 (q, 1H, aromat.);
7,01 - 7,09 (d, 1H, aromat.);
7,11 - 7,15 (m, 4H, aromat.);
7,33 - 7,38 (t, 1H, aromat.);
7,62 - 7,67 (m, 3H, aromat.);
8,18 (s, 1H, aromat.); 9,43 (s, 1H, OH)

### Beispiel 6

### N-Hydroxy-N-[5-[4-(1H-imidazol-1-yl)phenoxy]furan-2-yl-methyl]harnstoff

Die Herstellung erfolgte analog Beispiel 1, jedoch ohne Kupfer-I-bromid und ohne Kupferpulver. Anstelle von 4-Brombenzaldehyd wurden 35,3 g (0,25 Mol) 5-Nitrofuran-2-aldehyd eingesetzt. Die erhaltene Verbindung schmolz bei 155° C.
- ¹H-NMR (DMSO-d₆):: 4,42 (s, 2H, CH₂);
5,75 - 5,76 (d, 1H, aromat.);
6,30 - 6,31 (d, 1H, aromat.);
6,44 (s, 2H, NH₂);
7,09 (s, 1H, aromat.);
7,16 - 7,22 (m, 2H, aromat.);
7,62 - 7,67 (m, 3H, aromat.);
8,18 (s, 1H, aromat.);
9,44 (s, 1H, OH)

### Beispiel 7

### N-Hydroxy-N-[3-[4-(1H-imidazol-1-yl)phenoxy]-6-methoxyphenylmethyl]harnstoff

Die Herstellung erfolgte analog Beispiel 1. Anstelle von 4-Brombenzaldehyd wurden 54,3 g (0,25 Mol) 3-Brom-6-methoxy-benzaldehyd eingesetzt. Die erhaltene Verbindung schmolz bei 111° C.
- ¹H-NMR (DMSO-d₆):: 3,80 (s, 3H, OCH₃);
4,56 (s, 2H, CH₂);
6,41 (s, 2H, NH₂);
6,93 - 7,08 (m, 6H, aromat.);
7,55 - 7,63 (m, 3H, aromat.);
8,14 (s, 1H, aromat.);
9,50 (s, 1H, OH)

### Beispiel 8

### N-Hydroxy-N-{1-[4-[4-(1H-imidazol-1-yl)phenoxy]phenyl]ethyl}harnstoff

Die Herstellung erfolgte analog Beispiel 1. Anstelle von 4-Brombenzaldehyd wurden 49,8 g (0,25 Mol) 4-Bromacetophenon eingesetzt. Die Harnstoffverbindung wurde in Form farbloser Kristalle erhalten, die bei 160° C schmolzen.
- ¹H-NMR (DMSO-d₆):: 1,41 - 1,43 (d, 3H, CH₃);
5,28 - 5,36 (m, 1H, CH);
6,28 (s, 2H, NH₂);
6,90 - 7,16 (m, 5H, aromat.);
7,32 - 7,43 (d, 2H, aromat.);
7,52 - 7,68 (m, 3H, aromat.);
8,15 (s, 1H, aromat.); 9,07 (s, 1H, OH)

### Beispiel 9

### N-Hydroxy-N-[4-[4-(1H-imidazol-1-yl)phenoxy]phenylmethyl]-thioharnstoff

Das gemäß Beispiel 1 nach der Reduktion des Oxims mit Boran-Pyridin-Komplex erhaltene Hydroxylamin wurde isoliert und in 25 ml 1,4-Dioxan gelöst. Zu dieser Lösung wurden unter Rühren bei 20° C 1,7 ml Trimethylsilylisothiocyanat getropft. Nach 3 Stunden wurde mit einer gesättigten Lösung von Ammoniumchlorid und mit einer gesättigten Lösung von Natriumchlorid geschüttelt. Nach Trocknen der organischen Phasen über Natriumsulfat, Abdampfen der flüchtigen Bestandteile und Umkristallisieren des erhaltenen Rückstandes aus 1,4-Dioxan/Wasser wurden 1,92 g (56,4 % der Theorie) N-Hydroxy-N-[4-[4-(1H-imidazol-1-yl)phenoxy]phenylmethyl]thioharnstoff in Form farbloser Kristalle erhalten, die bei 174° C unter Zersetzung schmolzen.
- ¹H-NMR (DMSO-d₆):: 5,22 (s, 2H, CH₂);
7,02 - 7,15 (m, 5H, aromat.);
7,41 - 7,44 (d, 2H, aromat.);
7,62 - 7,69 (m, 3H, aromat.);
8,22 (s, 1H, aromat.); 10,08 (s, 1H, OH)

### Beispiel 10

### N-Hydroxy-N-[4-[3-(1H-imidazol-1-yl)phenoxy]phenylmethyl]harnstoff

Die Herstellung erfolgte analog Beispiel 1. Anstelle von 4-(1H-Imidazol-1-yl)phenol wurde 3-(1H-Imidazol-1-yl)phenol eingesetzt. Die Harnstoffverbindung wurde in Form farbloser Kristalle erhalten, die bei 158° C schmolzen.
- ¹H-NMR (DMSO-d₆):: 4,54 (s, 2H, CH₂); 6,37 (s, 2H, NH₂);
6,85 - 6,89 (d, 1H, aromat.);
7,00 - 7,10 (m, 3H, aromat.);
7,30 - 7,48 (m, 5H, aromat.);
7,71 (s, 1H, aromat.);
8,24 (s, 1H, aromat.);
9,42 (s, 1H, OH)

### Vergleichsbeispiel

### N-Hydroxy-N-[4-[2-(1H-imidazol-1-yl)ethoxy]phenylmethyl]harnstoff

6,11 g (0,05 Mol) 4-Hydroxybenzaldehyd wurden in 100 ml absolutem Dimethylformamid gelöst und mit 2,4 g (1 Äquivalent) einer 50 gewichtsprozentigen Natriumhydriddispersion in Paraffinöl in das Natriumsalz überführt. Anschließend wurde die erhaltene Suspension mit 376 g (2 Mol) 1,2-Dibromethan versetzt und 2 Stunden zum Sieden unter Rückfluß erhitzt. Nach dem Abkühlen auf 20° C wurden Salze und Dimethylformamid durch Ausschütteln mit Wasser entfernt und überschüssiges 1,2-Dibromethan bei 1,5 · 10³ Pa abdestilliert. Zu dem erhaltenen Rückstand wurde eine Natriumimidazolidsuspension, hergestellt aus 885 mg Imidazol und 624 mg 50 gewichtsprozentiger Natriumhydriddispersion in Paraffinöl und suspendiert in 20 ml absolutem Dimethylformamid, gegeben. Nach 5,5 Stunden bei 100° C wurde das Reaktionsgemisch in Eiswasser gegeben, angesäuert und zur Entfernung von Paraffinöl mit Dichlormethan extrahiert. Nach Einstellen eines pH-Wertes von 9 wurde der erhaltene 4-[2-(1H-Imidazol-1-yl)ethoxy]benzaldehyd mit Dichlormethan extrahiert. Nach Abdestillieren des Lösungsmittels wurden 2,14 g des Benzaldehydes als gelbes Öl erhalten.

Analog Beispiel 1 wurde der erhaltene Benzaldehyd in das Oxim überführt, dieses zum Hydroxylamin reduziert und daraus N-Hydroxy-N-[4-[2-(1H-imidazol-1-yl)ethoxy]phenylmethyl]harnstoff mit einem Zersetzungspunkt bei 155° C hergestellt.
- ¹H-NMR (DMSO-d₆):: 4,19 - 4,36 (m, 4H, CH₂CH₂);
4,43 (s, 2H, CH₂N);
6,31 (s, 2H, NH₂);
6,85 - 6,88 (m, 3H, aromat.);
7,18 - 7,23 (m, 3H, aromat.);
7,67 (s, 1H, aromat.);
9,32 (s, 1H, OH)

### Biologische Untersuchungen

Bei allen angegebenen IC₅₀-Werten handelt es sich um Absolutwerte.

RP-HPLC bedeutet Reversed-Phase-Hochleistungsflüssigkeitschromatographie.

Als stationäre Phase für die dünnschichtchromatographischen Trennungen wurde Silicagel Si 60 der Firma E. Merck, Darmstadt verwendet.

TXB₂ bedeutet Thromboxan B₂, HETE bedeutet Hydroxyeicosatetraensäure.

### Hemmung der 5-Lipoxygenase

### 1. Hemmung der 5-Lipoxygenase in intakten RBL-1-Zellen

Zur Ermittlung der 5-Lipoxygenasehemmung wurden basophile leukämische Leukozyten der Ratte (RBL-1-Zellen) in vitro gezüchtet, vom Nährboden abzentrifugiert, mit 0,05 molarem Kaliumphosphatpuffer (pH-Wert von 7,4) gewaschen und anschließend auf eine Zellzahl von 1 x 10⁷ Zellen pro ml eingestellt. Jeweils 1 ml dieser Zellsuspension wurde mit Indomethacin (10 µmol/l), Calciumchlorid (2 mmol/l) sowie entweder mit einer erfindungsgemäßen Verbindung (0,1 bis 100 µmol/l) oder mit Lösungsmittel (Ethanol und/oder Dimethylsulfoxid) bei Raumtemperatur (20° C) 3 Minuten vorinkubiert und anschließend mit radioaktiv markierter Arachidonsäure (20 µmol/l) und Calciumionophor A 23187 (20 µmol/l) 10 Minuten inkubiert.

Nach Abstoppen der Reaktion durch Zugabe von 20 µl Eisessig wurden die Reaktionsprodukte mit Essigsäureethylester extrahiert und mit Laufmittelgemisch dünnschichtchromatographisch getrennt (Jakschik et al., Biochem. Biophys. Res. Commun. 102, 624 (1981)).

Die Radioaktivitätsverteilung der verschiedenen Arachidonsäuremetabolite wurde mit Hilfe eines TLC-Linear-Analyzers gemessen. Aus den prozentualen Bildungsanteilen der 5-Lipoxygenaseprodukte bei Abwesenheit einer erfindungsgemäßen Verbindung sowie in Gegenwart verschiedener Konzentrationen an erfindungsgemäßen Verbindungen wurden graphisch aus halblogarithmischen Diagrammen die IC₅₀-Werte, d. h. die Konzentrationen, die eine 50 %ige Hemmung der 5-Lipoxygenase bewirken, bestimmt. Die Ergebnisse sind in der nachfolgenden Tabelle zusammengefaßt:

**Tabelle**

| erfindungsgemäße Verbindung hergestellt nach Beispiel | IC₅₀-Wert in µmolar |
|---|---|
| 1 | 2 |
| 2 | 5 |
| 3 | 5 |
| 4 | 3 |
| 5 | 6 |
| 10 | 2 |
| Vergleichsbeispiel | >100 |

### 2. Hemmung zellfreier 5-Lipoxygenase aus RBL-1-Zellen

Die zellfreie 5-Lipoxygenase wurde im 10.000 x g Überstand von RBL-1-Zellhomogenaten polarographisch gemessen (M. Haurand und L. Flohé, Biol. Chem. Hoppe-Seyler 369, 133 bis 142 (1988)). Nach 5-minütiger Vorinkubation mit Arachidonsäure (75 µmol/l), Adenosintriphosphat (4 mmol/l), reduziertem Glutathion (4 mmol/l) sowie mit einer erfindungsgemäßen Verbindung (0,1 bis 100 µmol/l) oder Lösungsmittel (Ethanol und/oder Dimethylsulfoxid) bei 35° C wurde die 5-Lipoxygenasereaktion durch Zugabe von Calciumchlorid (3 mmol/l) gestartet und simultan aufgezeichnet.

Die 5-Lipoxygenase-Aktivität wurde aus der Differenz der pO₂-Abnahme vor und nach Calciumchloridzugabe bestimmt. Bei Lag-Phasen wurde die Anfangsgeschwindigkeit der Reaktion nach der Lag-Phase ermittelt und die Dauer der Lag-Phase als zusätzlicher Parameter bestimmt. Die geprüften Substanzen zeigten im Konzentrationsbereich zwischen 5 und 10 µmol/l Lag-Phasen von 15 Sekunden.

Die ermittelten IC₅₀-Werte sind in der nachfolgenden Tabelle zu zusammengefaßt:

**Tabelle**

| erfindungsgemäße Verbindung hergestellt nach Beispiel | IC₅₀-Wert in µmolar |
|---|---|
| 1 | 7 |
| 3 | 3 |
| 5 | 2 |
| Vergleichsbeispiel | >100 |

### 3. Hemmung der 5-Lipoxygenase in Rattenvollblut nach oraler Applikation

Zur Charakterisierung der in-vivo-Verfügbarkeit der erfindungsgemäßen Verbindungen als 5-Lipoxygenasehemmer wurde ein von Tateson et al. in Brit. J. Pharmacol. 94, 528 (1988) beschriebenes Testmodell eingesetzt. Hierzu wurde männlichen Ratten (Stamm Wistar) eine erfindungsgemäße Verbindung peroral appliziert. Jeweils eine Stunde nach Applikation einer erfindungsgemäßen Verbindung wurde den Tieren nach letaler CO₂-Narkose unter Zusatz von Heparin als Antikoagulanz Vollblut entnommen. Aliquote des Vollblutes wurden im Wasserbad bei 37° C 30 Minuten mit dem Calciumionophor A 23187 in einer Endkonzentration von 15 µg/ml inkubiert. Anschließend wurden die Inkubationsansätze zentrifugiert und in aliquoten Teilen des zellfreien Plasmas die immunreaktiven Leukotrien B₄(iLTB₄)-Gehalte per Radio-Immun-Assay "RIA" (3H-LTB₄-RIA, Fa. Amersham) bestimmt. Der iLTB₄-Gehalt jeder Probe wurde anhand einer mit verdünntem Rattenplasma erstellten Standardkurve von LTB₄-Standardkonzentrationen rechnerisch ermittelt und als ng iLTB₄/ml Rattenplasma berechnet. Parallel zu den mit einer erfindungsgemäßen Verbindung behandelten Tieren wurden jeweils mit Vehikellösung peroral behandelte Tiere als Kontrollgruppe mitgeführt. Der Mittelwert aus den iLTB₄-Gehalten der Rattenplasmen dieser Kontrollgruppen diente als 100 %-Wert. Die Aktivität der ex-vivo iLTB₄-Synthese nach Applikation einer erfindungsgemäßen Verbindung in % wurde durch Division des Mittelwertes der iLTB₄-Gehalte in ng iLTB₄/ml der mit einer erfindungsgemäßen Verbindung behandelten Gruppe durch den Mittelwert der iLTB₄-Gehalte in ng iLTB₄/ml der Kontrollgruppe und anschließende Multiplikation mit dem Faktor 100 berechnet. Die jeweilige Hemmung in % wurde hieraus durch Subtrahieren der %-Aktivität von 100 ermittelt. Bei der Applikation einer Dosis von 21,5 mg/kg peroral ergaben sich für die erfindungsgemäßen Verbindungen die in der nachfolgenden Tabelle zusammengestellten Hemmwerte:

**Tabelle**

| erfindungsgemäße Verbindung hergestellt nach Beispiel | Hemmung in % |
|---|---|
| 1 | 89 |
| 2 | 95 |
| 3 | 91 |
| 4 | 93 |
| 6 | 84 |
| Vergleichsbeispiel | 42 |

### Hemmung der FMLP/Merthiolat-induzierten Leukotrien-Synthese von menschlichen polymorphkernigen Leukozyten

Human-Granulozyten wurden mittels Dextran-Sedimentation und Percoll-Gradientenzentrifugation isoliert und mit HBSS (Hanks buffered salt solution) auf eine Zellzahl von 1 x 10⁷/ml eingestellt. Die Zellen wurden bei 37° C 2 Minuten mit einer erfindungsgemäßen Verbindung oder als Kontrolle mit Lösungsmittel (Ethanol und/oder Dimethylsulfoxid) sowie weitere 2 Minuten mit Merthiolat (40 µmol/l) vorinkubiert und anschließend 15 Minuten mit FMLP (Formyl-Methionyl-Leucyl-Phenylalanin; 10⁻⁷mol/l) inkubiert. Die Arachidonsäuremetabolite wurden nach Festphasen-Extraktion mittels RP-HPLC analysiert und quantifiziert (M. Haurand und L. Flohé in Biochem. Pharmacol. 38, 2129 bis 2137 (1989)). Zur Auswertung wurden folgende Arachidonsäuremetabolite bestimmt: 5-HETE, LTB₄, 6-trans-LTB₄-Isomere, 20-OH-LTB₄ und 20-COOH-LTB₄.

Zur Untersuchung der Reversibilität der Hemmwirkung wurden die Zellen 5 Minuten bei 37° C mit einer erfindungsgemäßen Verbindung oder als Kontrolle mit Lösungsmittel (Ethanol und/oder Dimethylsulfoxid) inkubiert, 5 Minuten bei 300 x g zentrifugiert und in 2 ml HBSS resuspendiert. Anschließend wurden die Zellen 2 Minuten bei 37° C mit Merthiolat (40 µmol/l) vorinkubiert und 15 Minuten mit FMLP (10⁻⁷ mol/l) inkubiert.

Die in diesem Testmodell ermittelte reversible Hemmwirkung der nach Beispiel 1 hergestellten erfindungsgemäßen Verbindung betrug IC₅₀ = 2 µmolar.

### Hemmung der FMLP/Merthiolat-induzierten 12-HETE- und 5S, 12S-DiHETE-Synthese von menschlichen, polymorphkernigen Leukozyten und Thrombozyten

Coinkubationen von menschlichen Granulozyten und Thrombozyten wurden analog der Hemmung der FMLP/Merthiolat-induzierten Leukotriensynthese untersucht. In die Auswertung wurden zusätzlich 12-HETE und 5S,12S-Dihydroxy-HETE (5S,12S-DiHETE) mit einbezogen. Die nach Beispiel 1 hergestellte erfindungsgemäße Verbindung zeigte in diesem Testmodell keine Hemmwirkung gegenüber 12-Lipoxygenase.

### Hemmung der Cyclooxygenase

Schafssamenblasenmikrosomen (80 µg Protein pro ml Puffer; 50 mmolarer Kaliumphosphatpuffer , pH 7,4) wurden in 1 ml Aliquoten bei Raumtemperatur (20° C) 15 Minuten mit Arachidonsäure (20 µmol/l; 150000 dpm; 1-¹⁴C) sowie mit einer erfindungsgemäßen Verbindung (0,1 bis 100 µmol/l) oder Lösungsmittel (Ethanol und/oder Dimethylsulfoxid) inkubiert. Nach Zugabe von Essigsäure und Extraktion mit Essigsäureethylester wurden die Arachidonsäuremetabolite mittels Dünnschichtchromatographie in eine Prostaglandin- und eine Arachidonsäurefraktion getrennt. Die Auswertung mittels TLC-Linear-Analyzer basierte auf der Bestimmung der prozentualen Verteilung der ¹⁴C-markierten Fraktionen. Die IC₅₀-Werte der nach den Beispielen 1, 3 und 5 hergestellten erfindungsgemäßen Verbindungen betrugen >500 µmolar, das bedeutet, daß keine Cyclooxygenasehemmung vorlag.

### Hemmung der Thromboxansynthase

Human-Thrombozyten wurden nach Zugabe von Prostacyclin aus plättchenreichem Plasma isoliert und mit Tyrode-Hepes-Puffer (pH 7,4) auf eine Zellzahl von 2 x 10⁸/ml eingestellt. Die Zellen wurden in 1 ml Aliquoten bei 37° C 3 Minuten mit einer erfindungsgemäßen Verbindung (0,1 bis 100 µmol/l) oder mit Lösungsmittel (Ethanol und/oder Dimethylsulfoxid) als Kontrolle vorinkubiert und anschließend mit radioaktiv marktierter Arachidonsäure (3,5 µmol/l) 5 Minuten inkubiert. Durch Zugabe von Methanol, Chloroform und Ameisensäure wurde die Reaktion beendet, und gleichzeitig wurden die Arachidonsäuremetabolite extrahiert. Die Chloroformphase wurde unter Stickstoff zur Trockne eingeengt. Der Rückstand wurde in 50 µl Chloroform resuspendiert und dünnschichtchromatographisch analysiert. Die Radioaktivitätsverteilung der verschiedenen Arachidonsäuremetabolite wurde mit Hilfe eines TLC-Linear-Analyzers gemessen. Aus den prozentualen TXB₂-Bildungsanteilen der Kontrollen und bei verschiedenen Konzentrationen der erfindungsgemäßen Verbindungen wurden graphisch aus halblogarithmischen Diagrammen die IC₅₀-Werte bestimmt. Diese sind in der nachfolgenden Tabelle zusammengefaßt:

| erfindungsgemäße Verbindung hergestellt nach Beispiel | IC₅₀-Wert in µmolar |
|---|---|
| 1 | 2,8 |
| 3 | 2,9 |
| 5 | 2,5 |
| Vergleichsbeispiel | 4,0 |
| Zileuton (A 64077) | >100 |
| 4-[2-(1H-Imidazol-1-yl)ethoxy] benzoesäure, Hydrochlorid (UK 37248) | 2,7 |

Die nach den Beispielen 1, 3 und 5 hergestellten erfindungsgemäßen Verbindungen sind äquipotent mit dem Hydrochlorid der Imidazolylethoxybenzoesäure UK 37248, einem Thromboxansynthasehemmer, der jedoch 5-Lipoxygenase nicht hemmt. Der 5-Lipoxygenasehemmer Zileuton (A 64077), ein N-Hydroxyharnstoff, zeigte keine Hemmung der Thromboxansynthaseaktivität.

### Allergen-induzierte Bronchokonstriktion beim Meerschweinchen (Konzett-Rössler)

Die antiasthmatische Wirkung der erfindungsgemäßen Verbindungen wurde an narkotisierten, curarisierten und passiv beatmeten Meerschweinchen geprüft. Zur Auslösung einer asthmatischen Reaktion wurden die Tiere passiv mit IgE-haltigem anti-Ovalbumin-Serum (0,2 ml i.p.) sensibilisiert. Nach 48 Stunden wurde die anaphylaktische Bronchokonstriktion durch i.v.-Gabe von 0,2 mg/kg Ovalbumin als Allergen ausgelöst. Die sofort danach auftretende Bronchokonstriktion wurde nach einer modifizierten Methode nach Konzett und Rössler (Naunyn-Schmiedeberg's Arch. Exp. Path. Pharm. 195, 71 bis 74 (1940)) anhand des intratrachealen Druckanstiegs über einen Zeitraum von 15 Minuten gemessen. Effekte, die durch Histamin, Serotonin und sympathische Gegensteuerung ausgelöst werden, wurden durch intravenöse Vorbehandlung der Tiere mit 2,15 mg/kg Mepyramin, 46,4 µg/kg Propranolol, 4,64 mg/kg Atropin und 1 mg/kg Methysergid (5 Minuten vor Auslösung der Bronchokonstriktion durch das Allergen Ovalbumin) ausgeschaltet. Die erfindungsgemäßen Verbindungen wurden bei oraler Applikation eine Stunde vor der Allergengabe gegeben. Die gegenüber Kontrollversuchen (ohne Gabe einer erfindungsgemäßen Verbindung) ermittelten Abnahmen der Bronchokonstriktionen bei Applikation einer erfindungsgemäßen Verbindung sind in Form ihrer ED₄₀-Werte, d. h. der effektiven Dosen, bei der die Bronchokonstriktion im Mittel um 40 % gehemmt ist, in der nachfolgenden Tabelle zusammengestellt:

**Tabelle**

| erfindungsgemäße Verbindung hergestellt nach Beispiel | ED₄₀-Wert in mg/kg |
|---|---|
| 1 | 9,3 |
| 2 | 5,1 |
| 3 | 15,5 |
| 4 | 6,3 |

## Patentansprüche

1. 3- oder 4-(1H-Imidazol-1-yl)phenolderivate der allgemeinen Formel I 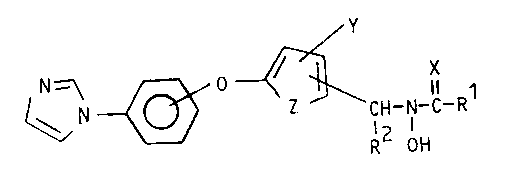 in der R¹ NH₂ oder CH₃, R² H oder CH₃, X O oder S, Y H, OH oder OCH₃ und Z -CH=CH-, O oder S bedeuten, und/oder deren Salze.

2. Imidazolylphenolderivate und/oder deren Salze nach Anspruch 1, dadurch gekennzeichnet, daß R² H bedeutet.

3. Imidazolylphenolderivate und/oder deren Salze nach einem oder beiden der Ansprüche 1 bis 2, dadurch gekennzeichnet, daß Z -CH=CH- bedeutet.

4. Imidazolylphenolderivate und/oder deren Salze nach einem oder beiden der Ansprüche 1 bis 2, dadurch gekennzeichnet, daß Z O bedeutet.

5. Imidazolylphenolderivate und/oder deren Salze nach einem oder beiden der Ansprüche 1 bis 2, dadurch gekennzeichnet, daß Z S bedeutet.

6. Imidazolylphenolderivate und/oder deren Salz nach einem oder mehreren der Ansprüche 1 bis 3, dadurch gekennzeichnet, daß das Imidazolylphenolderivat und/oder deren Salz N-Hydroxy-N-[4-[4-(1H-imidazol-1-yl)phenoxy]phenylmethyl]harnstoff und/oder das entsprechende Imidazoliumchlorid, N-Hydroxy-N-[3-[4-(1H-imidazol-1-yl)phenoxy]phenylmethyl]harnstoff, N-Hydroxy-N-[3-[4-(1H-imidazol-1-yl)phenoxy]-6-methoxy-phenylmethyl]harnstoff, N-Hydroxy-N-[4-[4-(1H-imidazol-1-yl)phenoxy]phenylmethyl]thioharnstoff oder N-Hydroxy-N-[4-[3-(1H-imidazol-1-yl)phenoxy)phenylmethyl]harnstoff ist.

7. Imidazolylphenolderivate und/oder deren Salze nach einem oder mehreren der Ansprüche 1 bis 3, dadurch gekennzeichnet, daß das Imidazolylphenolderivat N-Hydroxy-N-[4-[4-(1H-imidazol-1-yl)phenoxy]phenylmethyl]acetamid ist.

8. Imidazolylphenolderivate und/oder deren Salze nach Anspruch 1, dadurch gekennzeichnet, daß das Imidazolylphenolderivat N-Hydroxy-N-{1-[4-[4-(1H-imidazol-1-yl)-phenoxy]phenyl]ethyl}harnstoff ist.

9. Imidazolylphenolderivate und/oder deren Salze nach einem oder mehreren der Ansprüche 1, 2 und 4, dadurch gekennzeichnet, daß das Imidazolylphenolderivat N-Hydroxy-N-[5-[4-(1H-imidazol-1-yl)phenoxy]furan-2-yl-methyl]harnstoff ist.

10. Imidazolylphenolderivate und/oder deren Salze nach einem oder mehreren der Ansprüche 1, 2 und 5, dadurch gekennzeichnet, daß das Imidazolylphenolderivat N-Hydroxy-N-[5-[4-(1H-imidazol-1-yl)phenoxy]thien-2-yl-methyl]harnstoff ist.

11. Arzneimittel, dadurch gekennzeichnet, daß es als Wirkstoff wenigstens ein 3- oder 4-(1H-Imidazol-1-yl)phenolderivat und/oder wenigstens ein entsprechendes Salz gemäß den Ansprüchen 1 bis 10 enthält.

12. Arzneimittel nach Anspruch 11, dadurch gekennzeichnet, daß es zur topischen oder inhalativen Anwendung oder zur parenteralen Applikation geeignet ist.

13. Arzneimittel nach Anspruch 11, dadurch gekennzeichnet, daß es zur oralen Applikation geeignet ist.

14. Verfahren zur Herstellung von 3- oder 4-(1H-Imidazol-1-yl)phenolderivaten der allgemeinen Formel I 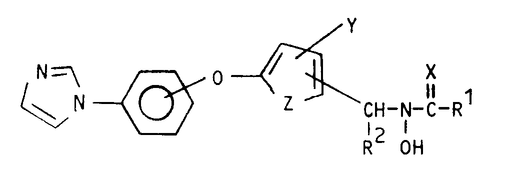 in der R¹ NH₂ oder CH₃, R² H oder CH₃, X O oder S, Y H, OH oder OCH₃ und Z -CH=CH-, O oder S bedeuten und/oder deren Salzen, dadurch gekennzeichnet, daß man eine Carbonylverbindung der allgemeinen Formel II
R³-Aryl
in der R³ Halogen, NO₂ oder OH und Aryl 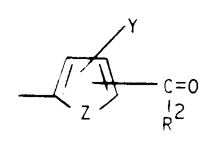 bedeuten, in Gegenwart eines Lösungsmittels, gegebenenfalls katalytischer Mengen an Kupfer und/oder Kupfer-I-salzen, und wenn R³=OH ist, nach Umsetzung mit einem Sulfochlorid, mit 3-(1H-Imidazol-1-yl)phenol oder 4-(1H-Imidazol-1-yl)phenol und/oder einem Alkalisalz eines dieser Phenole zu einer Verbindung der allgemeinen Formel III 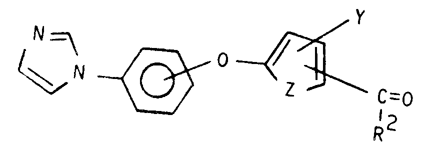 umsetzt, die Verbindung der allgemeinen Formel III in Gegenwart eines Lösungsmittels mit Hydroxylamin und/oder einem seiner Salze mit oder ohne Zusatz einer Base in das Oxim der allgemeinen Formel IV 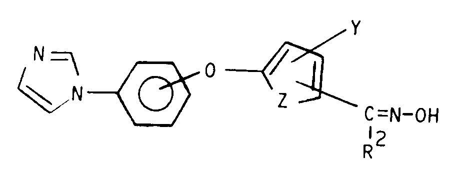 überführt, anschließend das erhaltene Oxim mit boranhaltigen Reduktionsmitteln in Gegenwart einer Säure zum Hydroxylamin der allgemeinen Formel V 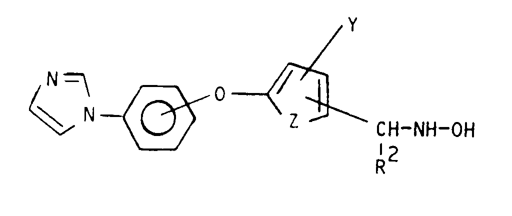 reduziert, das erhaltene Hydroxylamin in das Imidazolylphenolderivat der allgemeinen Formel I überführt entweder
A. durch Umsetzung mit einem Alkalicyanat oder einem Alkalithiocyanat oder
B. durch Umsetzung mit Trimethylsilylisocyanat oder Trimethylsilylisothiocyanat und anschließende Abspaltung der Trimethylsilylgruppe oder
C. durch Umsetzung mit einem Acetylierungsmittel und anschließende selektive basische Hydrolyse der O-Acetylgruppe der erhaltenen Bis-acetylverbindung
und danach gegebenenfalls aus dem Imidazolylphenylderivat der allgemeinen Formel I mit einer Säure ein Salz herstellt.

15. Verfahren nach Anspruch 14, dadurch gekennzeichnet, daß man eine Carbonylverbindung der allgemeinen Formel II, in der R³ Chlor, Brom oder Jod bedeutet, mit einem Alkalisalz von 3-(1H-Imidazol-1-yl)phenol oder 4-(1H-Imidazol-1-yl)phenol in Gegenwart katalytischer Mengen an Kupfer und/oder Kupfer-I-salzen zu einer Verbindung der allgemeinen Formel III umsetzt.

16. Verfahren nach einem oder beiden der Ansprüche 14 bis 15, dadurch gekennzeichnet, daß man als Carbonylverbindung der allgemeinen Formel II eine solche einsetzt, in der R² H bedeutet.

## Claims

1. 3- or 4-(1H-Imidazol-1-yl)phenol derivatives of the general formula I 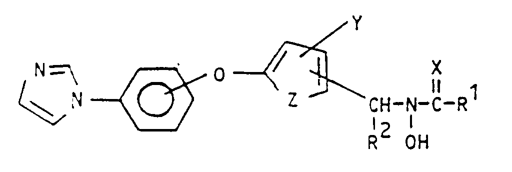 in which R¹ means NH₂ or CH₃, R² means H or CH₃, X means O or S, Y means H, OH or OCH₃ and Z means -CH=CH-, O or S, and/or the salts thereof.

2. Imidazolylphenol derivatives and/or the salts thereof according to claim 1, characterised in that R² means H.

3. Imidazolylphenol derivatives and/or the salts thereof according to one or both of claims 1 to 2, characterised in that Z means -CH=CH-.

4. Imidazolylphenol derivatives and/or the salts thereof according to one or both of claims 1 to 2, characterised in that Z means O.

5. Imidazolylphenol derivatives and/or the salts thereof according to one or both of claims 1 to 2, characterised in that Z means S.

6. Imidazolylphenol derivatives and/or the salts thereof according to one or more of claims 1 to 3, characterised in that the imidazolylphenol derivative and/or the salt thereof is N-hydroxy-N-[4-[4-(1H-imidazol-1-yl)phenoxy]phenylmethyl]urea and/or the corresponding imidazolium chloride, N-hydroxy-N-[3-[4-(1H-imidazol-1-yl)phenoxy]phenylmethyl]urea, N-hydroxy-N-[3-[4-(1H-imidazol-1-yl)phenoxy]-6- methoxyphenylmethyl]urea, N-hydroxy-N-[4-[4-(1H-imidazol-1-yl)phenoxy]phenylmethyl]thiourea or N-hydroxy-N-[4-[3-(1H-imidazol-1-yl)phenoxy]phenylmethyl]urea.

7. Imidazolylphenol derivatives and/or the salts thereof according to one or more of claims 1 to 3, characterised in that the imidazolylphenol derivative is N-hydroxy-N-[4-[4-(1H-imidazol-1-yl)phenoxy]phenylmethyl]acetamide.

8. Imidazolylphenol derivatives and/or the salts thereof according to claim 1, characterised in that the imidazolylphenol derivative is N-hydroxy-N-{1-[4-[4-(1H-imidazonl-1-yl)phenoxy]phenyl]ethyl}urea

9. Imidazolylphenol derivatives and/or the salts thereof according to one or more of claims 1, 2 and 4, characterised in that the imidazolylphenol derivative is N-hydroxy-N-[5-[4-(1H-imidazol-1-yl)phenoxy]furan2-ylmethyl]urea.

10. Imidazolylphenol derivatives and/or the salts thereof according to one or more of claims 1, 2 and 5, characterised in that the imidazolylphenol derivative is N-hydroxy-N-[5-[4-(1H-imidazol-1-yl)phenoxy]thien2-ylmethyl]urea.

11. Pharmaceutical, characterised in that it contains as active substance at least one 3- or 4-(1H-imidazol-1-yl)phenol derivative and/or at least one corresponding salt according to claims 1 to 10.

12. Pharmaceutical according to claim 11, characterised in that it is suitable for topical or inhalatory use or for parenteral administration.

13. Pharmaceutical according to claim 11, characterised in that it is suitable for oral administration.

14. Process for the production of 3- or 4-(1H-imidazol-1-yl)phenol derivatives of the general formula I 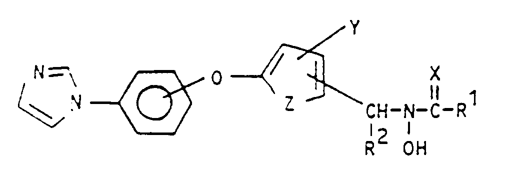 in which R¹ means NH₂ or CH₃, R² means H or CH₃, X means O or S, Y means H, OH or OCH₃ and Z means -CH=CH-, O or S, and/or the salts thereof, characterised in that a carbonyl compound of the general formula II
R³-aryl
in which R³ means halogen, NO₂ or OH and aryl means 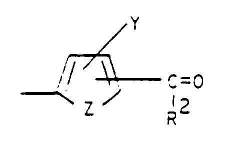 is reacted in the presence of a solvent, optionally catalytic quantities of copper and/or copper(I) salts and, if R³ = OH, after reaction with a sulfochloride, with 3-(1H-imidazol-1-yl)phenol or 4-(1H-imidazol-1-yl)phenol and/or an alkali metal salt of one of these phenols to yield a compound of the general formula III 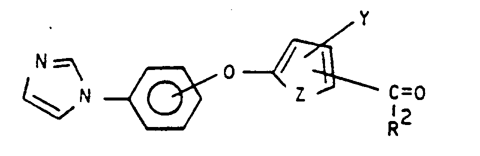 the compound of the general formula III is converted in the presence of a solvent with hydroxylamine and/or a salt thereof with or without addition of a base into the oxime of the general formula IV 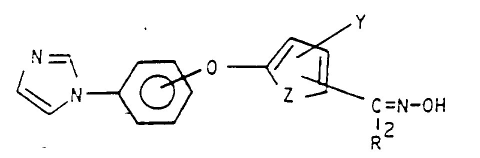 the resultant oxime is then reduced with a reducing agent containing borane in the presence of an acid to yield the hydroxylamine of the general formula V 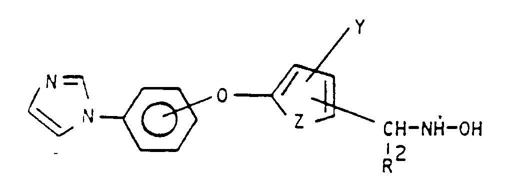 the resultant hydroxylamine is converted into the imidazolylphenol derivative of the general formula I either
A. by reaction with an alkali metal cyanate or an alkali metal thiocyanate or
B. by reaction with trimethylsilyl isocyanate or trimethylsilyl isothiocyanate with subsequent elimination of the trimethylsilyl group or
C. by reaction with an acetylating agent and subsequent selective basic hydrolysis of the O-acetyl group of the resultant bis-acetyl compound
and a salt is then optionally produced from the imidazolylphenol derivative of the general formula I with an acid.

15. Process according to claim 14, characterised in that a carbonyl compound of the general formula II, in which R³ means chlorine, bromine or iodine, is reacted with an alkali metal salt of 3-(1H-imidazol-1-yl)phenol or 4-(1H-imidazol-1-yl)phenol in the presence of catalytic quantities of copper and/or copper(I) salts to yield a compound of the general formula III.

16. Process according to one or both of claims 14 to 15, characterised in that the carbonyl compound of the general formula II used is one in which R² means H.

## Revendications

1. Dérivés de 3- ou 4-(1H-imidazole-1-yl)phénol de formule générale I 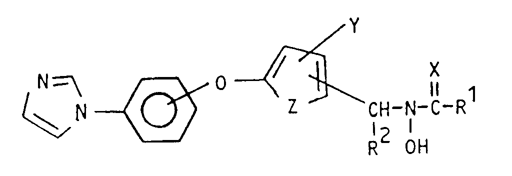 dans laquelle R¹ est un groupe NH₂ ou CH₃, R² est l'hydrogène ou un groupe CH₃, X est l'oxygène ou le soufre, Y est l'hydrogène, un groupe OH ou OCH₃ et Z est un groupe -CH=CH-, l'oxygène ou le soufre, et/ou leurs sels.

2. Dérivés d'imidazolylphénol et/ou leurs sels suivant la revendication 1, caractérisés en ce que R² désigne l'hydrogène.

3. Dérivés d'imidazolylphénol et/ou leurs sels suivant l'une des revendications 1 et 2 ou les deux, caractérisés en ce que Z est un groupe -CH=CH-.

4. Dérivés d'imidazolylphénol et/ou leurs sels suivant l'une des revendications 1 et 2 ou les deux, caractérisés en ce que Z est l'oxygène.

5. Dérivés d'imidazolylphénol et/ou leurs sels suivant l'une des revendications 1 et 2 ou les deux, caractérisés en ce que Z représente le soufre.

6. Dérivés d'imidazolylphénol et/ou leurs sels suivant une ou plusieurs des revendications 1 à 3, caractérisés en ce que le dérivé d'imidazolylphénol et/ou son sel est la N-hydroxy-N-[4-[4-(1H-imidazole-1-yl)phénoxy]phénylméthyl]urée et/ou le chlorure d'imidazolium correspondant, la N-hydroxy-N-[3-[4-(1H-imidazole-1-yl)phénoxy]phénylméthyl]-urée, la N-hydroxy-N-[3-[4-(1H-imidazole-1-yl)phénoxy]-6-méthoxy-phénylméthyl]urée, la N-hydroxy-N-[4-[4-(1H-imidazole-1-yl)-phénoxy]phénylméthyl]thiourée ou la N-hydroxy-N-[4-[3-(1H-imidazole-1-yl)phénoxy]phénylméthyl]urée.

7. Dérivés d'imidazolylphénol et/ou leurs sels suivant une ou plusieurs des revendications 1 à 3, caractérisés en ce que le dérivé d'imidazolylphénol est le N-hydroxyN-[4-[4-(1H-imidazole-1-yl)phénoxy]phénylméthyl]acétamide.

8. Dérivés d'imidazolylphénol et/ou leurs sels suivant la revendication 1, caractérisés en ce que le dérivé d'imidazolylphénol est la N-hydroxy-N-{1-[4-[4-(1H-imidazole-1-yl)phénoxy]phényl]éthyl}urée.

9. Dérivés d'imidazolylphénol et/ou leurs sels suivant une ou plusieurs des revendications 1, 2 et 4, caractérisés en ce que le dérivé d'imidazolylphénol est la N-hydroxy-N-[5-[4-(1H-imidazole-1-yl)phénoxy]furanne-2-yl-méthyl]urée.

10. Dérivés d'imidazolylphénol et/ou leurs sels suivant une ou plusieurs des revendications 1, 2 et 5, caractérisés en ce que le dérivé d'imidazolylphénol est la N-hydroxy-N-[5-[4-(1H-imidazole-1-yl)phénoxy]thién-2-ylméthyl]urée.

11. Médicament caractérisé en ce qu'il contient comme substance active au moins un dérivé de 3- ou 4-(1H-imidazole-1-yl)phénol et/ou au moins un sel correspondant suivant les revendications 1 à 10.

12. Médicament suivant la revendication 11, caractérisé en ce qu'il convient pour l'administration topique ou par inhalation ou pour l'administration parentérale.

13. Médicament suivant la revendication 11, caractérisé en ce qu'il convient pour l'administration orale.

14. Procédé de production de dérivés de 3- ou 4-(1H-imidazole-1-yl)phénol de formule générale I 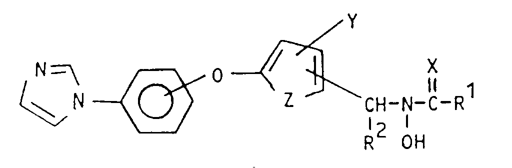 dans laquelle R¹ est un groupe NH₂ ou CH₃, R² est l'hydrogène ou un groupe CH₃, X est l'oxygène ou le soufre, Y est l'hydrogène, un groupe OH ou OCH₃ et Z est un groupe -CH=CH-, l'oxygène ou le soufre, et/ou de leurs sels, caractérisé en ce qu'on fait réagir un composé carbonylique de formule générale II
R³-aryle
dans laquelle R³ est un halogène, un groupe NO₂ ou OH et aryle représente un reste 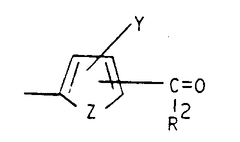 en présence d'un solvant, éventuellement de quantités catalytiques de cuivre et/ou de sels de cuivre-I et, lorsque R³ est un groupe OH, après réaction avec un sulfochlorure, avec le 3-(1H-imidazole-1-yl)phénol ou le 4-(1H-imidazole-1-yl)phénol et/ou un sel alcalin de l'un de ces phénols pour obtenir un composé de formule générale III 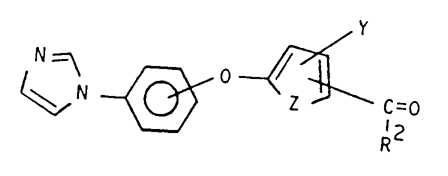 on transforme le composé de formule générale III, en présence d'un solvant, avec l'hydroxylamine et/ou l'un de ses sels, avec ou sans addition d'une base, en l'oxime de formule générale IV 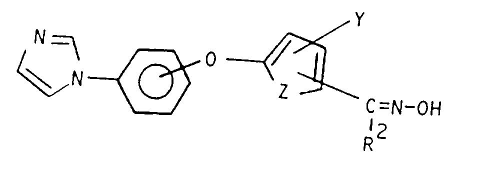 ensuite, on réduit l'oxime obtenue avec des agents réducteurs contenant un borane, en présence d'un acide, en l'hydroxylamine de formule générale V 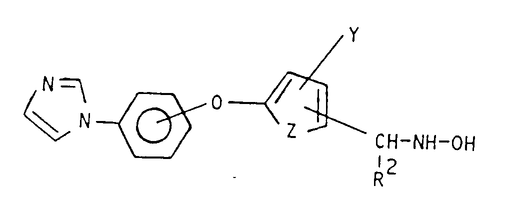 on transforme l'hydroxylamine obtenue en le dérivé d'imidazolylphénol de formule générale I,
A. par réaction avec un cyanate alcalin ou un thiocyanate alcalin, ou bien
B. par réaction avec l'isocyanate de triméthylsilyle ou l'isothiocyanate de triméthylsilyle, suivie de l'élimination du groupe triméthylsilyle, ou bien
C. par réaction avec un agent d'acétylation suivie d'une hydrolyse basique sélective du groupe O-acétyle du composé bis-acétylé obtenu
puis le cas échéant, on prépare un sel avec un acide à partir du dérivé d'imidazolylphénol de formule générale I.

15. Procédé suivant la revendication 14, caractérisé en ce qu'on fait réagir un composé carbonylique de formule générale II, dans laquelle R³ est le chlore, le brome ou l'iode, avec un sel alcalin de 3-(1H-imidazole-1-yl)phénol ou de 4-(1H-imidazole-1-yl)phénol en présence de quantités catalytiques de cuivre et/ou de sels de cuivre-I pour obtenir un composé de formule générale III.

16. Procédé suivant l'une des revendications 14 et 15 ou les deux, caractérisé en qu'on utilise comme composé carbonylique de formule générale II un composé carbonylique dans lequel R² représente l'hydrogène.
